(19) Europäisches Patentamt
European Patent Office
Office européen des brevets

(11) **EP 3 543 702 B1**

(12) **EUROPEAN PATENT SPECIFICATION**

(45) Date of publication and mention
of the grant of the patent:
**02.11.2022 Bulletin 2022/44**

(21) Application number: **18163573.1**

(22) Date of filing: **23.03.2018**

(51) International Patent Classification (IPC):
**G01N 33/68** [(2006.01)]

(52) Cooperative Patent Classification (CPC):
**G01N 33/6893**; G01N 2800/347; G01N 2800/54

(54) **METHODS FOR SCREENING A SUBJECT FOR THE RISK OF CHRONIC KIDNEY DISEASE AND COMPUTER-IMPLEMENTED METHOD**

VERFAHREN ZUM SCREENEN EINER PERSON AUF DAS RISIKO EINER CHRONISCHEN NIERENERKRANKUNG UND COMPUTERIMPLEMENTIERTES VERFAHREN

PROCÉDÉS DE CRIBLAGE D'UN SUJET POUR ÉVALUER LE RISQUE D'UNE MALADIE RÉNALE CHRONIQUE ET PROCÉDÉ MIS EN ŒUVRE PAR ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.09.2019 Bulletin 2019/39**

(73) Proprietors:
• **Roche Diabetes Care GmbH**
**68305 Mannheim (DE)**
Designated Contracting States:
**DE**
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**

(72) Inventors:
• **Petrich, Wolfgang**
**68305 Mannheim (DE)**
• **Huschto, Tony**
**68305 Mannheim (DE)**
• **Schneidinger, Bernd**
**68305 Mannheim (DE)**

(74) Representative: **Bittner, Thomas L.**
**Boehmert & Boehmert**
**Anwaltspartnerschaft mbB**
**Pettenkoferstrasse 22**
**80336 München (DE)**

(56) References cited:
**WO-A1-2015/140551      WO-A1-2016/170023**

• **ADLER PEROTTE ET AL: "Risk prediction for chronic kidney disease progression using heterogeneous electronic health record data and time series analysis", JOURNAL OF THE AMERICAN MEDICAL INFORMATICS ASSOCIATION, vol. 22, no. 4, 20 April 2015 (2015-04-20) , pages 872-880, XP055502840, AMSTERDAM, NL ISSN: 1067-5027, DOI: 10.1093/jamia/ocv024**
• **PAOLO FRACCARO ET AL: "An external validation of models to predict the onset of chronic kidney disease using population-based electronic health records from Salford, UK", BMC MEDICINE, vol. 14, no. 1, 12 July 2016 (2016-07-12), XP055502859, DOI: 10.1186/s12916-016-0650-2**
• **JUSTIN B. ECHOUFFO-TCHEUGUI ET AL: "Risk Models to Predict Chronic Kidney Disease and Its Progression: A Systematic Review", PLOS MEDICINE, vol. 9, no. 11, 20 November 2012 (2012-11-20), page e1001344, XP055503054, DOI: 10.1371/journal.pmed.1001344**
• **D. DUNKLER ET AL: "Risk Prediction for Early CKD in Type 2 Diabetes", CLINICAL JOURNAL OF THE AMERICAN SOCIETY OF NEPHROLOGY, vol. 10, no. 8, 14 July 2015 (2015-07-14), pages 1371-1379, XP055502568, ISSN: 1555-9041, DOI: 10.2215/CJN.10321014**

• VERGOUWE Y ET AL: "Progression to microalbuminuria in type 1 diabetes: development and validation of a prediction rule", DIABETOLOGIA, SPRINGER, BERLIN, DE, vol. 53, no. 2, 4 November 2009 (2009-11-04), pages 254-262, XP019776312, ISSN: 1432-0428

**Description**

The present invention refers to methods for screening a subject for the risk of chronic kidney disease and a computer-implemented method.

Background

In chronic kidney disease (CKD), kidney function is progressively lost, beginning with a decline in the glomerular filtration rate and/or albuminuria and progressing to end-stage renal disease. As a result, dialysis or renal transplant may be necessary (see Unger, J., Schwartz, Z., Diabetes Management in Primary Care, 2nd edition. Lippincott Williams & Wilkens, Philadelphia, USA, 2013). CKD is an serious problem, with an adjusted prevalence of 7% in 2013 (Glassock, R.J. et al., The global burden of chronic kidney disease: estimates, variability and pitfalls, Nat Rev Nephrol 13, 104-114, 2017). The early recognition of CKD could slow progression, prevent complications, and reduce cardiovascular-related outcomes (Platinga, L.C. et al., Awareness of chronic kidney disease among patients and providers, Adv Chronic Kidney Dis 17, 225-236, 2010). CKD may be a microvascular long-term complication of diabetes (Fioretto, P. et al., Residual microvascular risk in diabetes: unmet needs and future directions, Nat Rev Endocrinol 6, 19-25, 2010).

Algorithms for risk prediction of CKD by diabetic patients have been published, for example, by Dunkler et al. (Dunkler, D. et al., Risk Prediction for Early CKD in Type 2 Diabetes, Clin J Am Soc Nephrol 10, 1371-1379, 2015), Vergouwe et al. (Vergouwe, Y. et al., Progression to microalbuminuria in type 1 diabetes: development and validation of a prediction rule, Diabetologia 53, 254-262, 2010), Keane et al. (Keane, W.F. et al., Risk Scores for Predicting Outcomes in Patients with Type 2 Diabetes and Nephropathy: The RENAAL Study, Clin J Am Soc Nephrol 1, 761-767, 2006) and Jardine et al (Jardine, M.J. et al., Prediction of Kidney-Related Outcomes in Patients With Type 2 Diabetes, Am J Kidney Dis. 60, 770-778, 2012). Such published algorithms are derived from data originating from major clinical studies.

Further models for risk prediction of CKD have been described for example by Adler Perotte et al. (Adler Perotte et al.:"Risk prediction for chronic kidney disease progression using heterogeneous electronic health record data and time series analysis", Journal of the American Medical Informatics Association, vol. 22, no. 4, 20 April 2015 (2015-04-20), pages 872-880), Paolo Fraccaro et al. (Paolo Fraccaro et al.: "An external validation of models to predict the onset of chronic kidney disease using population-based electronic health records from Sal-ford, UK", BMC Medicine, vol. 14, no. 1, 12 July 2016 (2016-07-12), and Justin B. Echouffo-Tcheugui et al. (Justin B. Echouffo-Tcheugui et al.: "Risk Models to Predict Chronic Kidney Disease and Its Progression: A Systematic Review", Plos Medicine, vol. 9, no. 11, 20 November 2012 (2012-11-20), page e1001344).

Such predictive models based on clinical data represent an ideal setting with a preselected population, cross-checked and validated clinical data entries and often a narrow time window of observation. The outcomes therefore do not necessarily reveal the optimum pathways in terms of efficacy and effectiveness for a real-world population when inferred from clinical studies. In addition, most literature is focused on progression of diabetic nephropathy or CKD and therefore misses the early phase of this diabetic complication. Finally, patients are usually selected on the basis of a full set of respective features.

Summary

It is an object to provide improved methods for screening a subject for the risk of chronic kidney disease, allowing an early risk assessment for CKD based on real world data (RWD).

To solve this, methods for screening a subject for the risk of chronic kidney disease (CKD) according to the independent claims 1 and 15, respectively, are provided. Further, a computer-implemented method according to the independent claim 14 is provided. Further embodiments are disclosed in the dependent claims.

According to an aspect, a method for screening a subject for the risk of chronic kidney disease (CKD) is provided. The method comprises receiving marker data indicative for a plurality of marker parameters for a subject, such plurality of marker parameters indicating, for the subject for a measurement period, an age value, a sample level of creatinine, and a sample level of albumin; and determining a risk factor indicative of the risk of suffering CKD for the subject from the plurality of marker parameters. The determining comprises weighting the age value higher than the sample level of albumin, and weighting the sample level of creatinine higher than the sample level of albumin.

According to another aspect, a computer-implemented method for screening a subject for the risk of chronic kidney disease (CKD) in a data processing system is provided, the data processing system having a processor and a non-transitory memory storing a program causing the processor to execute:

- receiving marker data indicative for a plurality of marker parameters for a subject, such plurality of marker parameters indicating, for the subject for a measurement period, an age value, a sample level of albumin, and a sample level of creatinine ; and

- determining a risk factor indicative of the risk suffering CKD for the subject from the plurality of marker parameters, wherein the determining comprises
- weighting the age value higher than the sample level of albumin, and
- weighting the sample level of creatinine higher than the sample level of albumin.

According to a further aspect, a method for screening a subject for the risk of chronic kidney disease (CKD) is provided. The method comprises receiving marker data indicative for a plurality of marker parameters, such plurality of marker parameters indicating an age value for the subject, a sample level of creatinine for a measurement period, and a sample level of albumin for a measurement period; and determining a risk factor indicative of the risk of suffering CKD for the subject from the plurality of marker parameters. The determining comprises weighting the age value higher than the sample level of albumin, and weighting the sample level of creatinine higher than the sample level of albumin. At least one of the sample level of creatinine and the sample level of albumin is indicative of a generalized value of sample levels for a reference group of subjects not comprising the subject, for a respective measurement period of each subject of the reference group of subjects.

With regard to such method, for each subject of the reference group of subjects, the measurement period may be limited to two years and may end with a diabetes diagnosis of the respective subject of the reference group of subjects.

A generalized value of sample levels for a reference group of subjects not comprising the subject may be, for example, a maximum value, a minimum value, a mean value, a median value, or a slope determined for a plurality of sample levels for the respective measurement period of each subject of the reference group of subjects. The subjects of the reference group of subjects may be diabetes patients. For example, all subjects of the reference group of subjects may be diabetes patients.

The marker parameters may be indicative of real-world data which is not restricted regarding, for example, completeness or veracity of the data (unlike clinical data).

The age value for the subject for the measurement period may be an age value for the subject at the end of the measurement period.

Within the meaning of the present disclosure, weighting a first value or sample level higher than a second value or sample level means that the first value or sample level and the second value or sample level are used in an equation, such as an equation for determining a risk factor, in such a way that a relative change in the first value or sample level (for example a change of 10% in the first value) influences the result of the equation (for example the risk factor) more than the same relative change in the second value or sample level (in the example above, a change of 10% in the second value). For example, weighting may comcomprise multiplying the first value or sample level and the second value or sample level with appropriate respective constants. Depending on the expected first value or sample level and the expected second value or sample level and their respective units, weighting the first value or sample level higher than the second value or sample level may comprise multiplying the first value or sample level with a higher or smaller constant than the second value or sample level.

The method may further comprise the plurality of marker parameters indicating, for the subject, a blood sample level of creatinine. Thus, requesting the sample level of creatinine as a concentration in urine may be avoided. The plurality of marker parameters may indicate, for the subject, a selected blood sample level of creatinine selected from a plurality of blood sample levels of creatinine. For example, the selected blood sample level of creatinine may be a maximum value from the plurality of blood sample levels of creatinine. Alternatively or additionally, the plurality of marker parameters may indicate, for the subject, a calculated blood sample level of creatinine calculated from a plurality of blood sample levels of creatinine. For example, the calculated blood sample level of creatinine may be a statistical value calculated from the plurality of blood sample levels of creatinine, such as a mean value.

The sample level of creatinine may be provided in units of mg/dl (such as milligrams of creatinine per deciliter of blood).

The method may further comprise the plurality of marker parameters indicating, for the subject, a blood sample level of albumin. Thus, requesting the sample level of albumin as a concentration in urine may be avoided. The plurality of marker parameters may indicate, for the subject, a selected blood sample level of albumin selected from a plurality of blood sample levels of albumin. For example, the selected blood sample level of albumin may be a minimum value from the plurality of blood sample levels of albumin. Alternatively or additionally, the plurality of marker parameters may indicate, for the subject, a calculated blood sample level of albumin calculated from a plurality of blood sample levels of albumin. For example, the calculated blood sample level of albumin may be a statistical value calculated from the plurality of blood sample levels of albumin, such as a mean value.

The sample level of albumin may be provided in units of g/dl (such as grams of albumin per deciliter of blood).

The subject may be a diabetes patient. Thereby, the risk of chronic kidney disease in a diabetes patient may be screened.

Alternatively, all of the plurality of marker parameters may be for a subject for which a diabetes diagnosis is not available. For example, the subject may be at risk of becoming a diabetes patient. Thereby, the risk of chronic kidney disease in a subject not having been diagnosed with diabetes, for example a subject at risk of becoming a diabetes patient, may be screened. The receiving may comprise receiving marker data indicative for a plurality of marker parameters for the subject for which a diabetes diagnosis is not available.

The measurement period may be limited to two years. Thereby, values and/or sample levels of substances may be provided that have been collected within a time period of a maximum of two years with the risk factor indicating a risk of suffering CKD for the subject from the end of the measurement period onwards.

The subject may not have been diagnosed with diabetes by the end of the measurement period. For example, the risk of CKD may be screened in a subject that has recently been diagnosed with diabetes and the marker data may be indicative for a plurality of marker parameters for the subject for a measurement period that lies entirely before the diabetes diagnosis for the subject. Alternatively, the risk of CKD may be screened for a subject that has not been diagnosed with diabetes at all, the marker data therefore being indicative for a plurality of marker parameters for the subject for a measurement period in which the subject has not been diagnosed with diabetes.

The measurement period may lie after a diabetes diagnosis for the subject, at least in part. For example, at most 20% of the measurement period, preferably at most 10% of the measurement period, may lie after a time at which the subject was diagnosed with diabetes. For example, the subject may be a diabetes patient who has been diagnosed with diabetes for less than two years and the marker data may be indicative for a plurality of marker parameters for the patient for a measurement period, such as a measurement period of two years, that ends directly or shortly prior to the determining the risk factor, such that part of the plurality of marker parameters is for a time period before the diabetes diagnosis for the patient and part of the plurality of marker parameters is for a time period after the diabetes diagnosis for the patient.

The measurement period may lie entirely after a diabetes diagnosis for the diabetes patient. For example, the subject may be a diabetes patient who has been diagnosed with diabetes for more than two years and the marker data may be indicative for a plurality of marker parameters for the patient for a measurement period, such as a measurement period of two years, that ends directly or shortly prior to the determining the risk factor.

The risk factor may be indicative of the risk of suffering CKD for the subject within a prediction time period of three years from the end of the measurement period. The risk factor may be a probability for the subject of developing CKD within three years from the time the last value and/or sample level has been determined. Alternatively, the risk factor may be indicative of the risk of suffering CKD for the subject within a time period of less than three years, for example two years, from the end of the measurement period. As a further alternative, the risk factor may be indicative of the risk of suffering CKD for the subject within a time period of more than three years from the end of the measurement period.

The determining may further comprise weighting the age higher than the sample level of creatinine.

The receiving may comprise receiving marker data indicative for a plurality of marker parameters for a subject having a sample level of HbA1c of less than 6.5%. HbA1c is the C-fraction of glycated haemoglobin A1. The sample level of HbA1c may be provided in units of % (such as a percentage in blood). Alternatively, the sample level of HbA1c may be provided in units of mmol/mol (such as mmol of HbA1c per mol of blood).

The method may further comprise the plurality of marker parameters indicating, for the subject, a sample level of a glomerular filtration rate, and in the determining, weighting each of the age value, the sample level of albumin, and the sample level of creatinine higher than the sample level of a glomerular filtration rate.

The plurality of marker parameters may indicate, for the subject, a selected glomerular filtration rate selected from a plurality of glomerular filtration rates. For example, the selected glomerular filtration rate may be a minimum value from the plural glomerular filtration rates.

Alternatively or additionally, the plurality of marker parameters may indicate, for the subject, a calculated glomerular filtration rate calculated from a plurality of glomerular filtration rates. For example, the calculated glomerular filtration rate may be a statistical value calculated from the plurality of glomerular filtration rates, such as a mean value.

The glomerular filtration rate is known in the art to be indicative of the flow rate of filtered fluid through the kidney and is an important indicator for estimating renal function. The glomerular filtration rate may decrease due to renal disease. In embodiments, the glomerular filtration rate may be estimated using a Modification of Diet in Renal Disease (MDRD) formula, known in the art as such. For example, a MDRD formula using four variables relies on age, sex, ethnicity and serum creatinine of the subject for estimating glomerular filtration rate. In alternative embodiments, the glomerular filtration rate may be estimated using the CKD-EPI (Chronic Kidney Disease Epidemiology Collaboration) formula, known in the art as such. The CKD-EPI formula relies on age, sex, ethnicity and serum creatinine of the subject for estimating glomerular filtration rate. In further embodiments, the glomerular filtration rate may be estimated using other methods or may be directly determined. The sample glomerular filtration rate may be provided in units of ml/min/1.73m$^2$ (milliliters per minute per 1.73 square meters of body surface area).

The risk factor ($P_{CKD}$) may be determined according to the following equation:

$$P_{CKD} = \frac{e^{P_{CKD\_Pred}}}{1 + e^{P_{CKD\_Pred}} + e^{P_{Death\_Pred}}}$$

Herein, $P_{CKD\_Pred}$ may be calculated as

$$P_{CKD\_Pred} = c_{CKD1} \cdot age + c_{CKD2} \cdot creatinine + c_{CKD3} \cdot albumin + c_{CKD4}$$

and $P_{Death\_Pred}$ may be calculated as

$$P_{Death\_Pred} = c_{Death1} \cdot age + c_{Death2} \cdot creatinine + c_{Death3} \cdot albumin + c_{Death4},$$

wherein age is the age of the subject in years, creatinine is a sample level of creatinine for the subject, albumin is a sample level of albumin for the subject, and $c_{CKD1}$, $c_{CKD2}$, $c_{CKD3}$, $c_{CKD4}$, $c_{Death1}$, $c_{Death2}$, $c_{Death3}$ and $c_{Death4}$ are constants.
The sample level of creatinine may be a sample level of creatinine from a plurality of sample levels of creatinine. The sample level of albumin may be a sample level of albumin from a plurality of sample levels of albumin. The sample level of creatinine and/or the sample level of albumin may be a representative sample level from the respective plurality of sample levels of creatinine and/or albumin, such as a maximum sample level, a minimum sample level, a mean sample level and/or a median of the sample levels. In an exemplary embodiment, creatinine is a maximum sample level of creatinine from a plurality of sample levels of creatinine for the subject and albumin is a minimum sample level of albumin from a plurality of sample levels of albumin for the subject.
The constants $c_{CKD1}$, $c_{CKD2}$, $c_{CKD3}$, $c_{CKD4}$, $c_{Death1}$, $c_{Death2}$, $c_{Death3}$, and $c_{Death4}$ may be model specific constants. In embodiments, the constants $c_{CKD1}$, $c_{CKD2}$, $c_{CKD3}$, $c_{Death1}$, $c_{Death2}$ and $c_{Death3}$ may be constant weighting factors associated with the respective marker parameter.
In such embodiment, for example, the constants may be the following:

$c_{CKD1}$: 0.02739 /year;
$c_{CKD2}$: 1.387 dl/mg;
$c_{CKD3}$: - 0.3356 dl/g;
$c_{CKD4}$: - 3.1925;
$c_{Death1}$: 0.06103/year;
$c_{Death2}$: 0.8194 dl/mg;
$c_{Death3}$: - 0.9336 dl/g; and
$c_{Death4}$: - 3.3325.

In embodiments, any or each of the constants may be selected from a range of +/- 30% around such respective value, preferably from a range of +/- 20%, and more preferably from a range of +/- 10%
The risk factor ($P'_{CKD}$) may be determined according to the following equation:

$$P'_{CKD} = \frac{e^{P'_{CKD\_Pred}}}{1 + e^{P'_{CKD\_Pred}} + e^{P'_{Death\_Pred}}}$$

Herein, $P'_{CKD\_Pred}$ may be calculated as

$$P'_{CKD\_Pred} = c'_{CKD1} \cdot age + c'_{CKD2} \cdot creatinine + c'_{CKD3} \cdot albumin + c'_{CKD4} + c'_{CKD5} \cdot eGFR$$

and $P'_{Death\_Pred}$ may be calculated as

$$P'_{Death\_Pred} = c'_{Death1} \cdot age + c'_{Death2} \cdot creatinine + c'_{Death3} \cdot albumin + c'_{Death4} + c'_{Death5} \cdot eGFR \,,$$

wherein age is the age of the subject in years, creatinine is a sample level of creatinine for the subject, albumin is a sample level of albumin for the subject, eGFR is a sample level of estimated glomerular filtration rate for the subject, and $c'_{CKD1}$, $c'_{CKD2}$, $c'_{CKD3}$, $c'_{CKD4}$, $c'_{CKD5}$, $c'_{Death1}$, $c'_{Death25}$ $c'_{Death3}$, $c'_{Death4}$ and $c'_{Death5}$ are constants.

The sample level of creatinine may be a sample level of creatinine from a plurality of sample levels of creatinine. The sample level of albumin may be a sample level of albumin from a plurality of sample levels of albumin. The sample level of estimated glomerular filtration rate may be a sample level of estimated glomerular filtration rate from a plurality of sample levels of estimated glomerular filtration rate. The sample level of creatinine, the sample level of albumin and/or the sample level of estimated glomerular filtration rate may be a representative sample level from the respective plurality of sample levels of creatinine, albumin and/or estimated glomerular filtration rate, such as a maximum sample level, a minimum sample level, a mean sample level and/or a median of the sample levels. In an exemplary embodiment, creatinine is a maximum sample level of creatinine from a plurality of sample levels of creatinine for the subject, albumin is minimum a sample level of albumin from a plurality of sample levels of albumin for the subject and eGFR is a minimum sample level of estimated glomerular filtration rate from a plurality of sample levels of estimated glomerular filtration rate for the subject.

The constants $c'_{CKD1}$, $c'_{CKD2}$, $c'_{CKD3}$, $c'_{CKD4}$, $c'_{CKD5}$, $c'_{Death1}$, $c'_{Death2}$, $c'_{Death3}$, $c'_{Death4}$ and $c'_{Death5}$ may be model specific constants. In embodiments, the constants $c'_{CKD1}$, $c'_{CKD2}$, $c'_{CKD3}$, $c'_{CKD5}$, $c'_{Death1}$, $c'_{Death2}$, $c'_{Death3}$ and $c'_{Death5}$ may be constant weighting factors associated with the respective marker parameter.

In such embodiment, for example, the constants may be the following:

$c'_{CKD1}$: 0.02739 /year;
$c'_{CKD2}$: 1.387 dl/mg;
$c'_{CKD3}$: - 0.3356 dl/g;
$C'_{CKD4}$: - 1.3013;
$C'_{CKD5}$: - 0.02843 min·1.73m$^2$/ml;
$c'_{Death1}$: 0.06103 /year;
$c'_{Death2}$: 0.8194 dl/mg;
$C'_{Death3}$: - 0.9336 dl/g;
$c'_{Death4}$: - 4.4328; and
$c'_{Death5}$: 0.01654 min·1.73m$^2$/ml.

In embodiments, any or each of the constants may be selected from a range of +/- 30% around such respective value, preferably from a range of +/- 20%, and more preferably from a range of +/- 10%

In further embodiments, the risk factor ($P''_{CKD}$) may be determined according to the following equation:

$$P''_{CKD} = \frac{e^{P''_{CKD\_Pred}}}{1 + e^{P''_{CKD\_Pred}} + e^{P''_{Death\_Pred}}}$$

Herein, $P''_{CKD\_Pred}$ may be calculated as

$$P''_{CKD\_Pred} = c''_{CKD1} \cdot age + c''_{CKD2} \cdot creatinine + c''_{CKD3} \cdot albumin + c''_{CKD4} + c''_{CKD5} \cdot eGFR + c''_{CKD6} \cdot BMI + c''_{CKD7} \cdot Glucose + c''_{CKD8} \cdot HbA1c$$

and $P''_{Death\_Pred}$ may be calculated as

$$P''_{Death\_Pred} = c''_{Death1} \cdot age + c''_{Death2} \cdot creatinine + c''_{Death3} \cdot albumin + c''_{Death4} + c''_{Death5} \cdot eGFR + c''_{Death6} \cdot BMI + c''_{Death7} \cdot Glucose + c''_{Death8} \cdot HbA1c,$$

wherein age is the age of the subject in years, creatinine is a sample level of creatinine for the subject, albumin is a sample level of albumin for the subject, eGFR is a sample level of estimated glomerular filtration rate for the subject, BMI is a value of the Body Mass Index (BMI) for the subject, Glucose is a sample level of glucose for the subject, HbA1c is a sample level of C-fraction of glycated haemoglobin A1 for the subject and $c''_{CKD1}$, $c''_{CKD2}$, $c''_{CKD3}$, $c''_{CKD4}$, $c''_{CKD5}$,

$c''_{CKD6}$, $c''_{CKD7}$, $c''_{CKD8}$, $c''_{Death1}$, $c''_{Death2}$, $c''_{Death3}$, $c''_{Death4}$, $c''_{Death5}$, $c''_{Death6}$, $c''_{Death7}$ and $c''_{Death8}$ are constants. The BMI may be provided in units of kg/m$^2$ (kilograms per square meter) and determined as known in the art. The minimum sample level of glucose may be provided in units of mg/dl (such as milligrams of glucose per deciliter of blood).

The sample level of creatinine may be a sample level of creatinine from a plurality of sample levels of creatinine for the subject, the sample level of albumin may be a sample level of albumin from a plurality of sample levels of albumin for the subject, the sample level of estimated glomerular filtration rate may be a sample level of estimated glomerular filtration rate from a plurality of sample levels of estimated glomerular filtration rate for the subject, the value of the Body Mass Index (BMI) may be a value of the BMI from a plurality of values of the BMI for the subject, the sample level of glucose may be a sample level of glucose from a plurality of sample levels of glucose for the subject, and/or the sample level of C-fraction of glycated haemoglobin A1 may be a sample level of C-fraction of glycated haemoglobin A1 from a plurality of sample levels of C-fraction of glycated haemoglobin A1 for the subject

The sample level of creatinine, the sample level of albumin, the sample level of estimated glomerular filtration rate, the value of the Body Mass Index, the sample level of glucose, and/or the sample level of C-fraction of glycated haemoglobin A1 may be a representative sample level from the respective plurality of sample levels of creatinine, albumin, estimated glomerular filtration rate, Body Mass Index, glucose, and/or C-fraction of glycated haemoglobin A1, such as a maximum sample level, a minimum sample level, a mean sample level and/or a median of the sample levels. In an exemplary embodiment, creatinine is a maximum sample level of creatinine from a plurality of sample levels of creatinine for the subject, albumin is minimum a sample level of albumin from a plurality of sample levels of albumin for the subject, eGFR is a minimum sample level of estimated glomerular filtration rate from a plurality of sample levels of estimated glomerular filtration rate for the subject, .BMI is a minimum value of the Body Mass Index (BMI) from a plurality of values of the BMI for the subject, Glucose is a minimum sample level of glucose from a plurality of sample levels of glucose for the subject, and HbA is a mean sample level of C-fraction of glycated haemoglobin A1 from a plurality of sample levels of C-fraction of glycated haemoglobin A1 for the subject.

The constants $c''_{CKD1}$, $c''_{CKD2}$, $c''_{CKD3}$, $c''_{CKD4}$, $c''_{CKD5}$, $c''_{CKD6}$, $c''_{CKD7}$, $c''_{CKD8}$, $c''_{Death1}$, $c''_{Death2}$, $c''_{Death3}$, $c''_{Death4}$, $c''_{Death5}$, $c''_{Death6}$, $c''_{Death7}$ and $c''_{Death8}$ may be model specific constants. In embodiments, the constants $c''_{CKD1}$, $c''_{CKD2}$, $c''_{CKD3}$, $c''_{CKD5}$, $c''_{CKD6}$, $c''_{CKD7}$, $c''_{CKD8}$, $c''_{Death1}$, $c''_{Death2}$, $c''_{Death3}$, $c''_{Death5}$, $c''_{Death6}$, $c''_{Death7}$ and $c''_{Death8}$ may be constant weighting factors associated with the respective marker parameter.

In such embodiment, for example, the constants may be the following:

$c''_{CKD1}$: 0.02739 /year;
$c''_{CKD2}$: 1.387 dl/mg;
$c''_{CKD3}$: - 0.3356 dl/g;
$c''_{CKD4}$: - 2.409;
$c''_{CKD5}$: - 0.02843 min·1,73m$^2$/ml;
$c''_{CKD6}$: 0.01128 m$^2$/kg;
$c''_{CKD7}$: 0.0004946 dl/mg;
$c''_{CKD8}$: 0.0893 /%;
$c''_{Death1}$: 0.06103 /year;
$c''_{Death2}$: 0.8194 dl/mg;
$c''_{Death3}$: - 0.9336 dl/g;
$c''_{Death4}$: - 4.557;
$c''_{Death5}$: 0.01654 min·1.73m$^2$/ml;
$c''_{Death6}$: - 0.0101 m$^2$/kg;
$c''_{Death7}$: 0.0009107 dl/mg; and
$c''_{Death8}$: 0.04368/%.

In embodiments, any or each of the constants may be selected from a range of +/- 30% around such respective value, preferably from a range of +/- 20%, and more preferably from a range of +/- 10%

In embodiments, for any or all of creatinine, albumin, eGFR, BMI, Glucose and HbA, generalized values (creatinine$_{gen}$, albumin$_{gen}$, eGFR$_{gen}$, BMI$_{gen}$, Glucose$_{gen}$, HbA$_{gen}$) may be used instead of values for the subject. For example, mean values for the general population or mean values for a relevant sub-population may be used. As generalized values, mean values of representative values from a respective plurality of values for each population members may be used, for example mean values of a respective maximum value, a respective minimum value, a respective mean value and/or a respective median of values.

In such embodiments, for example, the generalized values may be the following:

creatinine$_{gen}$: 1.055 mg/dl;
albumin$_{gen}$: 3.835 g/dl;

eGFR$_{gen}$: 66.523 ml/min/1.73m$^2$;
BMI$_{gen}$: 32.295 kg/m$^2$;
Glucose$_{gen}$: 129.691 mg/dl; and
HbA$_{gen}$: 7.607%.

In embodiments, any or each of the generalized values may be selected from a range of +/-30% around such respective value, preferably from a range of +/- 20%, and more preferably from a range of +/- 10%

The method may further comprise determining a subject value recommendation and providing a recommendation output indicative of the subject value recommendation. The determining the subject value recommendation may comprise determining, based on the weighting of the marker parameters, a first marker parameter for which a generalized value was received and which is weighted higher than a second marker parameter for which a generalized value was received, and determining the subject value recommendation to be a recommendation to acquire a value for the first marker parameter for the subject. The recommendation output may be indicative of an instruction to acquire a value for the first marker parameter for the subject and re-perform the method for screening a subject for the risk of CKD, providing marker data comprising the value for the first marker parameter for the subject.

The method may comprise only determining the subject value recommendation and providing the recommendation output indicative of the subject value recommendation if it is determined that a value of accuracy of the risk factor is below an accuracy threshold. The value of accuracy of the risk factor may be determined based on for which marker parameters, generalized values are used. In embodiments, the value of accuracy of the risk factor may be determined in comparison to a reference risk factor that is determined using values for the subject for all or any of the marker parameters for which generalized values are used when determining the risk factor.

Within the meaning of the present disclosure, screening a subject for the risk of CKD means identifying a subject at risk of developing or having CKD.

A sample level in the sense of the present disclosure is a level of a substance, such as creatinine or albumin, in a sample of a bodily fluid of the subject. Sample levels may be determined in the same or different samples. Alternatively or additionally, for determining sample levels, measurements may be performed in the same or different samples. For example, a sample level of a substance may be determined from a plurality of measurements of the same substance in the same sample, for example by determining a mean value. In another example, at least one of a plurality of sample levels of the same substance may be determined in a first sample and at least another one of the plurality of sample levels of the same substance may be determined in a second sample. A sample level of a first substance and a sample level of a second substance may be determined in the same sample. Alternatively, a sample level of a first substance may be determined in a first sample and a sample level of a second substance may be determined in a second sample.

A computer program product may be provided, including a computer readable medium embodying program code executable by a process of a computing device or system, the program code, when executed, causing the computing device or system to perform the computer-implemented method for screening a subject for the risk of chronic kidney disease.

With regard to the computer-implemented method, the computer program product and the further method for screening a subject for the risk of chronic kidney disease, the alternative embodiments described above may apply *mutatis mutandis.*

In the computer-implemented method, the sample level of albumin may be a sample level of albumin in a bodily fluid sample and the sample level of creatinine may be a sample level of creatinine in another bodily fluid.

In the computer-implemented method, the program may further cause the processor to execute generating output data indicative of the risk factor and outputting the output data to an output device of the data processing system. The output device may be any device suitable for outputting the output data, for example a display device of the data processing system, such as a monitor, and/or a transmitter device for transmitting for wired and/or wireless data transmission. The output data may be output to a user, for example a physician. The output data may be output via a display of the data processing system.

The data processing system may comprise a plurality of data processing devices, each data processing device having a processor and a memory. The marker data may be provided in a first data processing device. For example, the marker data may be received in the first data processing device by user input via an input device and/or by data transfer. The marker data may be sent from the first data processing device to a second data processing device which may be located remotely with respect to the first data processing device. The marker data may be received in the second data processing device and the risk factor may then be determined in the second data processing device. Result data indicative of the risk factor may be sent from the second data processing device to the first data processing device or, alternatively or additionally, to a third data processing device. The result data may then be stored in the first and/or the third data processing device and/or output via an output device of the first and/or the third data processing device.

The first data processing device and/or the third data processing device may be a local device, such as a client computer, and the second data processing device may be a remote device, such as a remote server.

9

Alternatively, the functionality of at least the first data processing device and the second data processing device may be provided in the same data processing device, for example a computer, such as a computer in a physician's office. All steps of the computer-implemented method may be executed in the same data-processing device.

Brief description of the figures

In the figures show:

Fig. 1 the distribution of age in an example teaching training set, validation set and further validation set;
Fig. 2 the distribution of HbA1C in an example teaching training set, validation set and further validation set;
Fig. 3 a comparison of algorithms for predicting CKD;
Fig. 4 a comparison of algorithms for predicting CKD using subcohorts;
Fig. 5 another comparison of algorithms for predicting CKD; and
Fig. 6 a further comparison of algorithms for predicting CKD.

Description of further embodiments

Following, embodiments are described by way of example.

In general, in any of the embodiments of the method for screening a subject for the risk of CKD, $creatinine_{max}$ may be a maximum sample level of creatinine from a plurality of sample levels of creatinine for the subject, $albumin_{min}$ may be a minimum sample level of albumin from a plurality of sample levels of albumin for the subject, $eGFR_{min}$ may be a minimum sample level of estimated glomerular filtration rate from a plurality of sample levels of estimated glomerular filtration rate for the subject, $BMI_{min}$ may be a minimum value of the Body Mass Index (BMI) from a plurality of values of the BMI for the subject, $Glucose_{min}$ may be a minimum sample level of glucose from a plurality of sample levels of glucose for the subject and $HbA_{mean}$ may be a mean sample level of C-fraction of glycated haemoglobin A1 from a plurality of sample levels of C-fraction of glycated haemoglobin A1 for the subject. Such values and/or sample levels may be determined from values and/or sample levels already on file for the subject. Alternatively or in addition, values and/or sample levels may be determined for the subject specifically for use with the method for screening a subject for the risk of CKD. Values and/or sample levels may be real world data, i.e., unlike clinical data, they may not be restricted regarding, for example, completeness or veracity of the data.

In any of the embodiments of the method for screening a subject for the risk of CKD, $creatinine_{max}$ may be expressed in units of mg/dl, $albumin_{min}$ may be expressed in units of g/dl, $eGFR_{min}$ may be expressed in units of ml/min/1.73m$^2$, $BMI_{min}$ may be expressed in units of kg/m$^2$, $Glucose_{min}$ may be a expressed in units of mg/dl and $HbA_{mean}$ may be expressed in units of %. Glomerular filtration rates may be estimated using an MDRD formula, known in the art as such. Alternatively, glomerular filtration rates may be estimated using the CKD-EPI formula, known in the art as such.

In an embodiment, marker data is received for a subject suffering from diabetes. In alternative embodiments, the subject does not suffer from diabetes but may is at risk of suffering from diabetes in the future. The marker data is indicative for marker parameters age, $creatinine_{max}$ and $albumin_{min}$ for the subject. The parameter "age" indicates the age of the subject in years. The parameter "$creatinine_{max}$" is indicative of a maximum sample level of creatinine from a plurality of sample levels of creatinine on file for the subject and collected over the prior 2 years from blood samples. The parameter "$albumin_{mm}$" is indicative of a minimum sample level of albumin from a plurality of sample levels of albumin on file for the subject and collected over the prior 2 years from blood samples.

A risk factor indicative of the risk of suffering CKD for the subject is determined from the plurality of marker parameters according to the following equations:

$$P_{CKD} = \frac{e^{P_{CKD\_Pred}}}{1 + e^{P_{CKD\_Pred}} + e^{P_{Death\_Pred}}}$$

$$P_{CKD\_Pred} = 0.02739 \cdot age \,/\, year + 1.387 \cdot creatinine_{max} \cdot dl/mg$$
$$- 0.3356 \cdot albumin_{min} \cdot dl/g - 3.1925$$

$$P_{Death\_Pred} = 0.06103 \cdot age \,/\, year + 0.8194 \cdot creatinine_{max} \cdot dl/mg$$
$$- 0.9336 \cdot albumin_{min} \cdot dl/g - 3.3325$$

Thereby, the age value is weighted higher than the sample level of albumin and the sample level of creatinine is weighted higher than the sample level of albumin.

In another embodiment, marker data is received for a subject suffering from diabetes. In alternative embodiments, the subject does not suffer from diabetes but may is at risk of suffering from diabetes in the future. The marker data is indicative for marker parameters age, $creatinine_{max}$, $albumin_{min}$ and $eGFR_{min}$ for the subject. The parameter "age" indicates the age of the subject in years. The parameter "$creatinine_{max}$" is indicative of a maximum sample level of creatinine from a plurality of sample levels of creatinine on file for the subject and collected over the prior 2 years from blood samples. The parameter "$albumin_{min}$" is indicative of a minimum sample level of albumin from a plurality of sample levels of albumin on file for the subject and collected over the prior 2 years from blood samples. The parameter "$eGFR_{min}$" is indicative of a minimum sample level of estimated glomerular filtration rate from a plurality of sample levels of estimated glomerular filtration rate on file for the subject and collected over the prior 2 years.

A risk factor indicative of the risk of suffering CKD for the subject is determined from the plurality of marker parameters according to the following equations:

$$P_{CKD} = \frac{e^{P_{CKD\_Pred}}}{1 + e^{P_{CKD\_Pred}} + e^{P_{Death\_Pred}}}$$

$$P_{CKD\_Pred} = 0.02739 \cdot age\,/\,year + 1.387 \cdot creatinine_{max} \cdot dl/mg$$
$$- 0.3356 \cdot albumin_{min} \cdot dl/g - 0.02843 \cdot eGFR_{min} \cdot min \cdot 1.73m^2/ml - 1.3013$$

$$P_{Death\_Pred} = 0.06103 \cdot age\,/\,year + 0.8194 \cdot creatinine_{max} \cdot dl/mg$$
$$- 0.9336 \cdot albumin_{min} \cdot dl/g + 0.01654 \cdot eGFR_{min} \cdot min \cdot 1.73m^2/ml - 4.4328$$

Thereby, the age value is weighted higher than the sample level of albumin, the sample level of creatinine is weighted higher than the sample level of albumin and each of the age value, the sample level of albumin, and the sample level of creatinine are weighted higher than the sample level of glomerular filtration rate.

In a further embodiment, marker data is received for a subject suffering from diabetes. In alternative embodiments, the subject does not suffer from diabetes but may is at risk of suffering from diabetes in the future. The marker data is indicative for marker parameters age, $creatinine_{max}$, $albumin_{min}$, $eGFR_{min}$, $BMI_{min}$, $Glucose_{min}$ and $HbA_{mean}$ for the subject. The parameter "age" indicates the age of the subject in years. The parameter "$creatinine_{max}$" is indicative of a maximum sample level of creatinine from a plurality of sample levels of creatinine on file for the subject and collected over the prior 2 years from blood samples. The parameter "$albumin_{min}$" is indicative of a minimum sample level of albumin from a plurality of sample levels of albumin on file for the subject and collected over the prior 2 years from blood samples. The parameter "$eGFR_{min}$" is indicative of a minimum sample level of estimated glomerular filtration rate from a plurality of sample levels of estimated glomerular filtration rate on file for the subject and collected over the prior 2 years. The parameter "$BMI_{min}$" is indicative of a minimum value for the Body Mass Index from a plurality of values for the Body Mass Index on file for the subject and collected over the prior 2 years. The parameter "$Glucose_{min}$" is indicative of a minimum sample level of blood glucose from a plurality of sample levels of blood glucose on file for the subject and collected over the prior 2 years. The parameter "$HbA_{mean}$" is indicative of a mean sample level of C-fraction of glycated haemoglobin A1 from a plurality of sample levels of C-fraction of glycated haemoglobin A1 on file for the subject and collected over the prior 2 years.

A risk factor indicative of the risk of suffering CKD for the subject is determined from the plurality of marker parameters according to the following equations:

$$P_{CKD} = \frac{e^{P_{CKD\_Pred}}}{1 + e^{P_{CKD\_Pred}} + e^{P_{Death\_Pred}}}$$

$$P_{CKD\_Pred} = 0.02739 \cdot age\,/\,year + 1.387 \cdot creatinine_{max} \cdot dl/mg - 0.3356 \cdot albumin_{min} \cdot dl/g$$
$$- 0.02843 \cdot eGFR_{min} \cdot min \cdot 1.73m^2/ml + 0.01128 \cdot BMI_{min}$$
$$+ 0.0004946 \cdot Glucose_{min} \cdot dl/mg + 0.0893 \cdot HbA_{mean}\,/\% - 2.409$$

$$P_{Death\_Pred} = 0.06103 \cdot age / year + 0.8194 \cdot creatinine_{max} \cdot dl/mg - 0.9336 \cdot albumin_{min} \cdot dl/g$$
$$+ 0.01654 \cdot eGFR_{min} \cdot min \cdot 1.73m^2/ml - 0.0101 \cdot BMI_{min}$$
$$+ 0.0009107 \cdot Glucose_{min} \cdot dl/mg + 0.04368 \cdot HbA_{mean} /\% - 4.557$$

Thereby, the age value is weighted higher than the sample level of albumin, the age is weighted higher than the sample level of creatinine, the sample level of creatinine is weighted higher than the sample level of albumin and each of the age value, the sample level of albumin, and the sample level of creatinine are weighted higher than the sample level of glomerular filtration rate. Further, each of the age value, the sample level of albumin, the sample level of creatinine and the sample level of glomerular filtration rate are weighted higher than each of the value of the Body Mass Index, the sample level of of blood glucose and the sample level of C-fraction of glycated haemoglobin A1.

In embodiments of the method for screening a subject for the risk of CKD, all or any of the values to be multiplied with the values and/or sample levels for the subject in determining $P_{CKD\_Pred}$ and/or $P_{Death\_Pred}$ may be determined as follows:

An algorithm is taught using electronic health record (HER) data, for example from 417,912 people with diabetes (types 1 and 2) among more than 55 million people represented in a database. The data is retrieved for the time window starting 2 years before the initial diagnosis of diabetes and lasting until up to 3 years following this diagnosis. The data can be considered as real-world data (RWD) and no general restrictions on, for example, completeness or veracity of the data are applied. Missing data is imputed with the cohort's mean value before feature selection and teaching the algorithm. Logistic regression is chosen for teaching rather than a black box approach such as deep learning. This may allow for the medical interpretation of the data-driven analysis. After teaching, an independent sample set of data, for example originating from 104,504 further individuals in the same database, is used for independent validation. In addition, the algorithm is applied to data, for example from 82,912 persons with type-2 diabetes included in a further database.

ICD codes may be used as target variables for training as well as the CKD reference diagnosis in the analysis of the validation results. The definition of the target feature "CKD" may be solely based on the occurrence of the respective ICD codes in the databases. In order to maintain the RWD character of the data set, no additions or changes may be made to the databases. Such ICD codes may comprise ICD-9 codes and ICD-10 codes, for example the following ICD codes: 250.40, 250.41, 250.42, 250.43, 585.1, 585.2, 585.3, 585.4, 585.5, 585.6, 585.9, 403.00, 403.01, 403.11, 403.90, 403.91, 404.0, 404.00, 404.01, 404.02, 404.03, 404.1, 404.10, 404.11, 404.12, 404.13, 404.9, 404.90, 404.91, 404.92, 404.93, 581.81, 581.9, 583.89, 588.9, E10.2, E10.21, E10.22, E10.29, E11.2, E11.21, E11.22, E11.29, N17.0, N17.1, N17.2, N17.8, N17.9, N18.1, N18.2, N18.3, N18.4, N18.5, N18.6, N18.9, N19, I12.0, I12.9, I13, 113.0, I13.1, I13.10, I13.11, I13.2, N04.9, N05.8, N08 and/or N25.9.

In an embodiment, the ICD-9 codes 250.40, 403.90, 585.3, 585.9 are the most abundant diagnosis in the respective time windows of the data set and they occur in > 5% of the cases within each of the data sets.

In further embodiments of the method for screening a subject for the risk of CKD, all or any of the values to be multiplied with the values and/or sample levels for the subject in determining $P_{CKD\_Pred}$ and/or $P_{Death\_Pred}$ may be determined as follows:

In order to allow an early risk assessment for CKD, EHR data is extracted from a database, which includes longitudinal data originating from more than 55 million patients with thousands of person-specific features. The data extracted from the database for the investigation originates from 522,416 people newly diagnosed with diabetes. The data is retrieved for the time window starting 2 years before the initial diagnosis of diabetes and lasting until up to 3 years following this diagnosis. People with prior renal dysfunctions are excluded in order to perform an unbiased risk assessment for the later development of CKD. Following the guidelines for the diagnosis of diabetes, it is requested that the concentration of the β-N-1-deoxyfructosyl component of hemoglobin (HbA1C), an important clinical laboratory parameter in diabetes diagnosis and treatment, was determined at least once prior to (or within 7 days after) the initial diagnosis of diabetes. The data selected from the database can be considered as RWD because no further restrictions on the completeness or veracity of the data are applied. In order to cope with these challenges arising from the use of RWD the following approach may be implemented:

1. The data selected from the database is randomly split into a teaching set (417,912 people) and a validation set (104,504 people).

2. Features is selected on the basis of a data-driven correlation analysis within the teaching set and cross-checked for conceptual (especially medical) relevance.

3. Missing values are imputed with the dataset's mean value.

4. The risk predictor is taught exclusively in this RWD's teaching set.

5. After the teaching is completed, the validation set is subjected to the algorithm in order to assess the quality of the algorithm. No further readjustment of the algorithm is performed.

6. In addition, RWD from 82,912 people represented in a further database is used as a further, independent validation set.

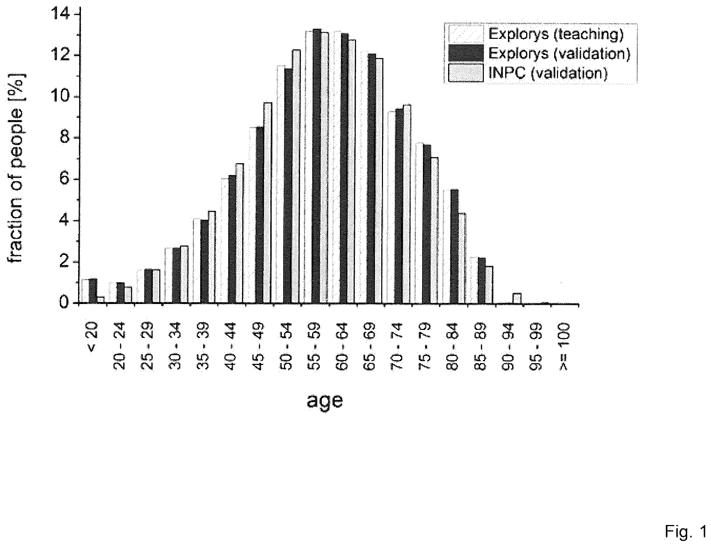

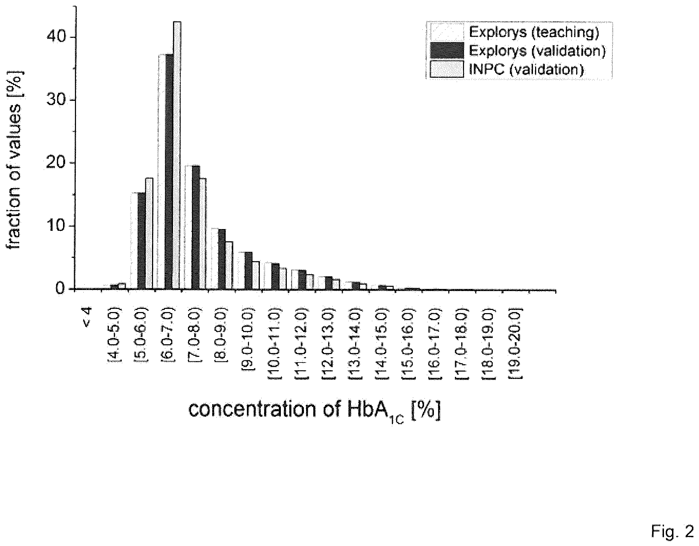

In an example teaching training set (from the IBM Explorys database; see Kaelber, D.C. et al., Patient characteristics associated with venous thromboembolic events: a cohort study using pooled electronic health record data, J Am Med Inform Assoc 19, 965-972, 2012), validation set (from the IBM Explorys database) and further validation set (from the Indiana Network for Patient Care (INPC); see McDonald, C. J. et al., The Indiana Network for Patient Care: a working local health information infrastructure, Health Affairs 24, 1214-1220, 2005), 50.7%, 50.9% and 51.7% of the persons, respectively, are female. The median age of each population is 60 years, 60 years, and 59 years, respectively. The median concentrations of HbA1C are 6.8%, 6.8%, and 6.6%, respectively. The distributions of age and HbA1C are shown in Fig. 1 and 2, respectively.

In certain embodiments, for feature selection, almost 300 features are initially chosen based on medical as well as data-driven criteria. This feature set is then culled in multiple steps. Observational features that are defined for less than half of the patients in the cohort are removed, as are outliers of continuous features. Categorical features with $\geq$ 99% of occurrences in a single category and continuous features with a standard deviation of $\leq 0.001\%$ are not considered. Finally, only those features which already showed correlation with the diagnosis of CKD in a univariate analysis as quantified by Pearson's chi-squared coefficient $\chi^2 > 0.95$ are retained. For predictive analysis, a logistic regression model based on forward selection (see Bursac, Z. et al., Purposeful selection of variables in logistic regression, Source code for biology and medicine 3, 17, 2008; and Hosmer Jr., D.W. et al., Applied logistic regression, Vol. 398, John Wiley & Sons, 2013) is trained on the teaching set and delivers the person's age, body mass index, glomerular filtration rate and the concentrations of glucose, albumin, and creatinine as the most prominent parameters. An assessment of the medical relevance of these features may be performed to ensure clinical applicability, in contrast to a "black box" approach based on, for example, deep learning. HbA1c may be added to the top-7 feature list in order to reflect current state-of-the-art methods. The teaching of algorithms may be based on correlation, but may not infer any causality. After teaching, the algorithm is applied to the two independent datasets, namely the validation sets.

ICD codes may be used as target variables for training as well as the CKD reference diagnosis in the analysis of the validation results. The definition of the target feature "CKD" may be solely based on the occurrence of the respective ICD codes in the databases. In order to maintain the RWD character of the data set, no additions or changes may be made to the databases. Such ICD codes may comprise ICD-9 codes and ICD-10 codes, for example the following ICD codes: 250.40, 250.41, 250.42, 250.43, 585.1, 585.2, 585.3, 585.4, 585.5, 585.6, 585.9, 403.00, 403.01, 403.11, 403.90, 403.91, 404.0, 404.00, 404.01, 404.02, 404.03, 404.1, 404.10, 404.11, 404.12, 404.13, 404.9, 404.90, 404.91, 404.92, 404.93, 581.81, 581.9, 583.89, 588.9, E10.2, E10.21, E10.22, E10.29, E11.2, E11.21, E11.22, E11.29, N17.0, N17.1, N17.2, N17.8, N17.9, N18.1, N18.2, N18.3, N18.4, N18.5, N18.6, N18.9, N19, 112.0, 112.9, I13, I13.0, 113.1, I13.10, I13.11, I13.2, N04.9, N05.8, N08 and/or N25.9.

In an embodiment, the ICD-9 codes 250.40, 403.90, 585.3, 585.9 are the most abundant diagnosis in the respective time windows of the data set and they occur in > 5% of the cases within each of the data sets.

Experimental data

The area under the receiver operating characteristic (compare Swets, J.A., Measuring the accuracy of diagnostic systems, Science 240, 1285-1293, 1988) curve (AUC) is frequently used to measure the quality of clinical markers as well as machine learning algorithms (see Bradley, A.P., The use of the area under the ROC curve in the evaluation of machine learning algorithms, Pattern Recognition 30, 1145-1159, 1997). A perfect marker would achieve AUC=1.0, whereas flipping a coin would result in AUC=0.5. After teaching the model according to the present disclosure using the seven most promising features, the AUC of the prediction algorithm amounted to 0.7937 (0.790...0.797) when applied to the overall independent validation data (Explorys: 0.761, INPC: 0.831). The AUC increased to 0.7939 and 0.7967 if the top-10 and top-12 features were used for evaluation, respectively. In turn, a simple HbA1C model (see The Diabetes Control and Complications Trial Research Group. The effect of intensive treatment of diabetes on the development and progression of long-term complications in insulin-dependent diabetes mellitus, N Engl J Med 329, 977-986, 1993) yielded 0.483 (0.477...0.489) for the same datasets. The algorithm according to the present disclosure therefore outperforms risk predictors using HbA1C alone for people newly diagnosed with diabetes.

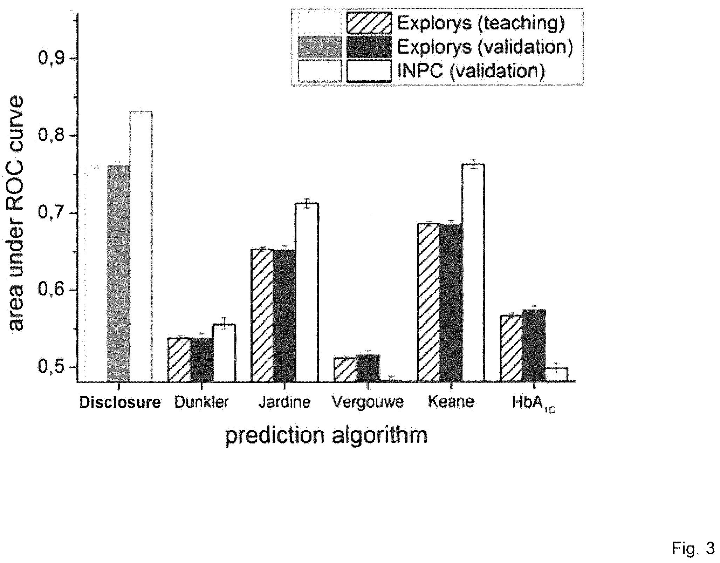

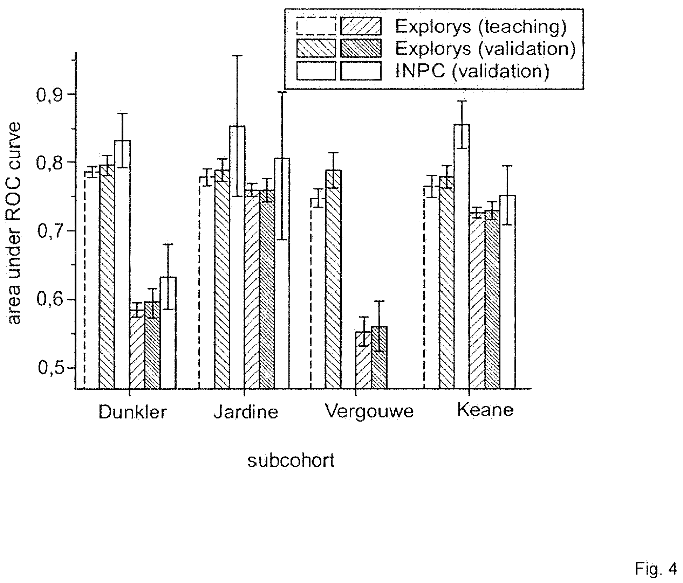

In further analysis, the algorithm according to the present disclosure was compared to published algorithms derived from data sourced from major clinical studies such as the ONTARGET, ORIGIN, RENAAL and ADVANCE studies (cf. Dunkler, D. et al., Risk Prediction for Early CKD in Type 2 Diabetes, Clin J Am Soc Nephrol 10, 1371-1379, 2015; Vergouwe, Y. et al., Progression to microalbuminuria in type 1 diabetes: development and validation of a prediction rule, Diabetologia 53, 254-262, 2010; Keane, W.F. et al., Risk Scores for Predicting Outcomes in Patients with Type 2 Diabetes and Nephropathy: The RENAAL Study, Clin J Am Soc Nephrol 1, 761-767, 2006; and Jardine, M.J. et al., Prediction of Kidney-Related Outcomes in Patients With Type 2 Diabetes, Am J Kidney Dis. 60, 770-778, 2012). As shown in Fig. 3, the algorithm according to the present disclosure outperformed each of these algorithms for all RWD cohorts. While this finding is important in terms of applicability and relevance in everyday settings, it may be argued that the validity of the published algorithms is limited to the inclusion and exclusion criteria of the corresponding clinical studies. Therefore, subcohorts of the IBM Explorys and INPC databases were formed according to the selection criteria of these studies, and the algorithm according to the present disclosure (without any retraining) was benchmarked against the literature algorithms solely for these subcohorts. Although the AUCs of the published algorithms increased for all specific subcohorts as expected, the superiority of the RWD-trained model according to the present disclosure prevailed (Fig. 4). However, the inclusion and exclusion criteria for the subcohorts could not be met precisely in all cases for the present RWD set because the clinical studies demanded some information which is not available in the database (e.g. waist-to-hip ratio). In addition, there were differences in the choice of the complication incidence time window. Nevertheless, the features that were prioritized for classification with the algorithm according to the present disclosure are similar those reported in the literature, thus further bolstering the algorithm's validity.

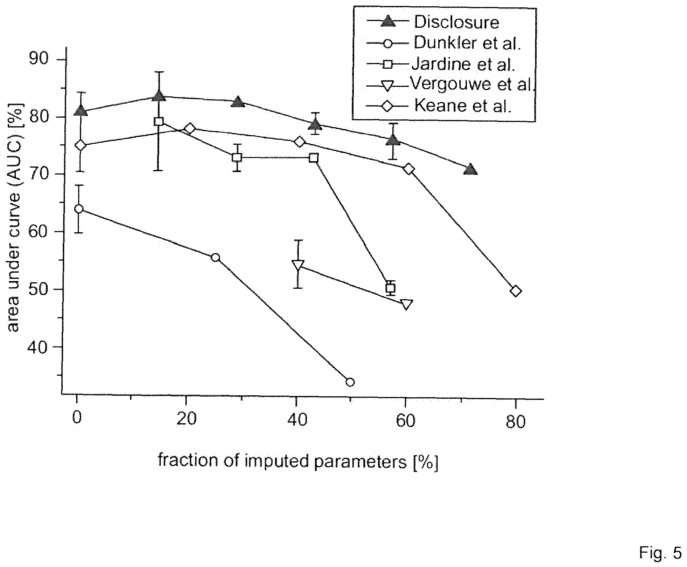

The use of RWD and in particular the inclusion of incomplete or erroneous data in the training set for the algorithm according to the present disclosure constitutes a major difference compared to clinical study-based algorithms. The imputation of missing data provides a typical example of predictive analytics in RWD cohorts, whereas imputation would be inconceivable in a clinical study setting. To further elucidate the role of imputation, the algorithm according to the present disclosure was applied to RWD solely representing individuals providing a complete set of information (i.e. no imputation was necessary). In this case, the AUCs remained comparable to the previous values for the overall RWD set, that is 0.792 (0.787...0.797), 0.791 (0.780...0.801), and 0.809 (0.769... 0.846) for the Explorys teaching training set, the Explorys validation set, and the INPC validation set, respectively. Further analysis revealed the rapid loss of classification accuracy with an increasing fraction of imputed data when the earlier algorithms were tested, whereas the algorithm according to the present disclosure achieved much higher stability, even for higher proportions of imputed data (Fig. 5). It is concluded that - at least in the present example - the teaching training of predictive analytics algorithms using RWD could achieve equivalent or even enhanced accuracy compared to clinical trial data, but further testing on additional datasets will be necessary before these conclusions can be generalised.

In summary, it is demonstrated that a predictive algorithm for CKD performed significantly better in individuals newly diagnosed with diabetes if trained on RWD rather than clinical study data. This statement held true when the algorithm according to the present disclosure was applied to the overall RWD cohort as well as specific subcohorts as defined by the corresponding clinical studies. The results support the path towards high-quality predictive models that can be applied in a clinical setting, enabling the shift towards personalized and outcome-based healthcare.

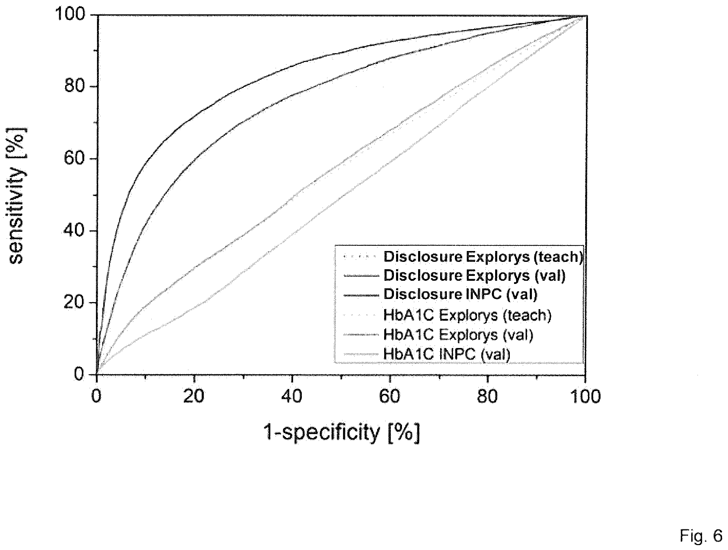

The performance of a method for screening a subject for the risk of CKD or for identifying those people at high risk of developing CKD may be judged according to sensitivity (fraction of correctly predicted high-risk patients) and specificity (fraction of correctly assigned low-risk patients). However, either of these numbers can be improved at the expense of the other simply by changing the threshold between high and low risk. Hence, data pairs of sensitivity and specificity may be illustrated in forms of the so-called receiver operating characteristic (ROC) curve (see Swets, J.A., Measuring the accuracy of diagnostic systems, Science 240, 1285-1293, 1988) in which the sensitivity is plotted as a function of 1-specificity (which corresponds to the fraction of falsely assigned high-risk persons). The ROC curve of the risk model according to the present disclosure is shown for the Explorys training set, the Explorys validation set and the INPC validation set in Fig. 6 together with the corresponding ROC curves for a model based solely on HbA1C.

For a perfect classifier, the ROC curve reaches the upper-left corner. In fact, the threshold corresponding to the data pair closest to this corner is dubbed the "optimal threshold". When aiming for high sensitivity, an alternative threshold may be chosen to guarantee a sensitivity of, for example, 90%. The corresponding results are summarized in the following Table together with the positive predictive value (PPV) and negative predictive value (NPV). Similar measures from the field of bioinformatics - namely accuracy and F-score (Van Rijsbergen, C.J., Information Retrieval, Butterworth-Heinemann Newton, MA, USA, 1979) - supplement the list of examples in the Table.

| | | Cohort | sensitivity | specificity | PPV | NPV | acc. | F-measure |
|---|---|---|---|---|---|---|---|---|
| a) | HbA1c | Explorys (teach) | 53.5 | 55.1 | 11.7 | 91.4 | 55.0 | 19.2 |
| | | Explorys (val) | 54.4 | 55.2 | 11.9 | 91.6 | 55.1 | 19.5 |
| | | INPC (val) | 37.5 | 61.5 | 11.3 | 88.2 | 58.7 | 17.4 |

(continued)

| | | Cohort | sensitivity | specificity | PPV | NPV | acc. | F-measure |
|---|---|---|---|---|---|---|---|---|
| b) | present model | Explorys (teach) | 68.2 | 72.6 | 21.7 | 95.4 | 72.1 | 32.9 |
| | | Explorys (val) | 68.3 | 72.4 | 21.6 | 95.3 | 72.0 | 32.8 |
| | | INPC (val) | 79.3 | 71.2 | 26.6 | 96.3 | 72.2 | 39.8 |
| c) | present model* | Explorys (teach) | (90.0) | 35.0 | 13.3 | 96.9 | 40.5 | 23.2 |
| | | Explorys (val) | 90.0 | 34.9 | 13.3 | 96.9 | 40.4 | 23.2 |
| | | INPC (val) | 95.3 | 27.6 | 14.7 | 97.8 | 35.5 | 25.5 |

A comparative evaluation of the full algorithm according to the present disclosure (seven values / sample levels for the subject, missing values / sample levels imputed) to a reduced algorithm according to the present disclosure (age, $creatinine_{max}$ and $albumin_{min}$ for the subject, population mean values for the remaining values / sample levels), respectively applied to INPC data, has resulted in an AUC of 0.831 (confidence interval 0.827 to 0.836) for the full algorithm and an AUC of 0.823 (confidence interval 0.818 to 0.827) for the reduced algorithm. Therefore, even with the reduced algorithm, useful predictions may be achieved.

**Claims**

1. A method for screening a subject for the risk of chronic kidney disease (CKD), comprising

   - receiving marker data indicative for a plurality of marker parameters for a subject, such plurality of marker parameters indicating, for the subject for a measurement period, an age value, a sample level of creatinine, and a sample level of albumin; and
   - determining a risk factor indicative of the risk of suffering CKD for the subject from the plurality of marker parameters, wherein the determining comprises

      - weighting the age value higher than the sample level of albumin, and
      - weighting the sample level of creatinine higher than the sample level of albumin.

2. The method according to claim 1, further comprising the plurality of marker parameters indicating, for the subject, a blood sample level of creatinine.

3. The method according to claim 1 or 2, further comprising the plurality of marker parameters indicating, for the subject, a blood sample level of albumin.

4. The method according to at least one of the preceding claims, wherein the subject is a diabetes patient.

5. The method according to at least one of the preceding claims, wherein the measurement period is limited to two years.

6. The method according to at least one of the preceding claims, wherein the subject has not been diagnosed with diabetes by the end of the measurement period.

7. The method according claim 4 or 5, wherein the measurement period lies after a diabetes diagnosis for the subject, at least in part.

8. The method according to at least one of the preceding claims, wherein the risk factor is indicative of the risk of suffering CKD for the subject within a prediction time period of three years from the end of the measurement period.

9. The method according to at least one of the preceding claims, wherein the determining further comprises weighting the age higher than the sample level of creatinine.

10. The method according to at least one of the preceding claims, wherein the receiving comprises receiving marker data indicative for a plurality of marker parameters for a subject having a sample level of HbA1c of less than 6.5%.

**11.** The method according to at least one of the preceding claims, further comprising

- the plurality of marker parameters indicating, for the subject, a sample level of a glomerular filtration rate; and
- in the determining, weighting each of the age value, the sample level of albumin, and the sample level of creatinine higher than the sample level of a glomerular filtration rate.

**12.** The method according to at least one of claims 1 to 10, wherein the risk factor is determined according to the equation

$$P_{CKD} = \frac{e^{P_{CKD\_Pred}}}{1 + e^{P_{CKD\_Pred}} + e^{P_{Death\_Pred}}},$$

and wherein

- $P_{CKD}$ is the risk factor;
- $P_{CKD\_Pred} = c_{CKD1} \cdot age + c_{CKD2} \cdot creatinine + c_{CKD3} \cdot albumin + c_{CKD4}$ ;
- $P_{Death\_Pred} = c_{Death1} \cdot age + c_{Death2} \cdot creatinine + c_{Death3} \cdot albumin + c_{Death4}$ ;
- age is the age of the subject;
- creatinine is a sample level of creatinine for the subject;
- albumin is a sample level of albumin for the subject; and
- $c_{CKD1}$, $c_{CKD2}$, $c_{CKD3}$, $c_{CKD4}$, $c_{Death1}$, $c_{Death2}$, $c_{Death3}$ and $c_{Death4}$ are constants.

**13.** The method according to at least one of claims 1 to 11, wherein the risk factor is determined according to the equation

$$P'_{CKD} = \frac{e^{P'_{CKD\_Pred}}}{1 + e^{P'_{CKD\_Pred}} + e^{P'_{Death\_Pred}}},$$

and wherein

- $P'_{CKD}$ is the risk factor;
-

$$P'_{CKD\_Pred} = c'_{CKD1} \cdot age + c'_{CKD2} \cdot creatinine + c'_{CKD3} \cdot albumin + c'_{CKD4} + c'_{CKD5} \cdot eGFR ;$$

-

$$P'_{Death\_Pred} = c'_{Death1} \cdot age + c'_{Death2} \cdot creatinine + c'_{Death3} \cdot albumin + c'_{Death4} + c'_{Death5} \cdot eGFR ;$$

- age is the age of the subject;
- creatinine is a sample level of creatinine for the subject;
- albumin is a sample level of albumin for the subject;
- eGFR is a sample level of estimated glomerular filtration rate for the subject; and
- $c'_{CKD1}$, $c'_{CKD2}$, $c'_{CKD3}$, $c'_{CKD4}$, $c'_{CKD5}$, $c'_{Death1}$, $c'_{Death2}$, $c'_{Death3}$, $c'_{Death4}$ and $c'_{Death5}$ are constants.

**14.** A computer-implemented method for screening a subject for the risk of chronic kidney disease (CKD) in a data processing system having a processor and a non-transitory memory storing a program causing the processor to execute:

- receiving marker data indicative for a plurality of marker parameters for a subject, such plurality of marker parameters indicating, for the subject for a measurement period, an age value, a sample level of albumin, and a sample level of creatinine; and
- determining a risk factor indicative of the risk suffering CKD for the subject from the plurality of marker parameters, wherein the determining comprises

- weighting the age value higher than the sample level of albumin, and
- weighting the sample level of creatinine higher than the sample level of albumin.

**15.** A method for screening a subject for the risk of chronic kidney disease (CKD), comprising

- receiving marker data indicative for a plurality of marker parameters, such plurality of marker parameters indicating

- an age value for the subject,
- a sample level of creatinine for a measurement period, and
- a sample level of albumin for a measurement period; and

- determining a risk factor indicative of the risk of suffering CKD for the subject from the plurality of marker parameters, wherein the determining comprises

- weighting the age value higher than the sample level of albumin, and
- weighting the sample level of creatinine higher than the sample level of albumin,

wherein
- at least one of the sample level of creatinine and the sample level of albumin is indicative of a generalized value of sample levels for a reference group of subjects not comprising the subject, for a respective measurement period of each subject of the reference group of subjects.


## Patentansprüche

**1.** Verfahren zum Screening eines Subjektes auf das Risiko einer chronischen Nierenerkrankung (CKD), umfassend

- Empfangen von Markerdaten, die für eine Mehrzahl von Markerparametern für ein Subjekt kennzeichnend sind, wobei die derartige Mehrzahl von Markerparametern für das Subjekt für einen Messzeitraum einen Alterswert, einen Probenspiegel von Kreatinin und einen Probenspiegel von Albumin anzeigen; und
- Bestimmen eines Risikofaktors, der auf das Risiko, an CKD zu erkranken, für das Subjekt aus der Mehrzahl von Markerparametern hinweist, wobei das Bestimmen Folgendes umfasst

- Gewichten des Alterswertes höher als den Probenspiegel von Albumin, und
- Gewichten des Probenspiegels von Kreatinin höher als den Probenspiegel von Albumin.

**2.** Verfahren nach Anspruch 1, ferner umfassend die Mehrzahl von Markerparametern, die für das Subjekt einen Blutprobenspiegel von Kreatinin anzeigen.

**3.** Verfahren nach Anspruch 1 oder 2, ferner umfassend die Mehrzahl von Markerparametern, die für das Subjekt einen Blutprobenspiegel von Albumin anzeigen.

**4.** Verfahren nach mindestens einem der vorhergehenden Ansprüche, wobei das Subjekt ein Diabetes-Patient ist.

**5.** Verfahren nach mindestens einem der vorhergehenden Ansprüche, wobei der Messzeitraum auf zwei Jahre begrenzt ist.

**6.** Verfahren nach mindestens einem der vorhergehenden Ansprüche, wobei bei dem Subjekt bis zum Ende des Messzeitraums kein Diabetes diagnostiziert worden ist.

**7.** Verfahren nach Anspruch 4 oder 5, wobei der Messzeitraum mindestens teilweise nach einer Diabetes-Diagnose bei dem Subjekt liegt.

**8.** Verfahren nach mindestens einem der vorhergehenden Ansprüche, wobei der Risikofaktor das Risiko angibt, dass das Subjekt innerhalb eines Vorhersagezeitraums von drei Jahren nach dem Ende des Messzeitraums an CKD erkrankt.

9. Verfahren nach mindestens einem der vorhergehenden Ansprüche, wobei das Bestimmen ferner ein Gewichten des Alters höher als den Probenspiegel des Kreatinins umfasst.

10. Verfahren nach mindestens einem der vorhergehenden Ansprüche, wobei das Empfangen ein Empfangen von Markerdaten umfasst, die für eine Mehrzahl von Markerparametern für ein Subjekt, das einen HbA1c-Probenspiegel von weniger als 6,5 % aufweist, kennzeichnend sind.

11. Verfahren nach mindestens einem der vorhergehenden Ansprüche, ferner umfassend

- die Mehrzahl von Markerparametern, die für das Subjekt einen Probenspiegel einer glomerulären Filtrationsrate anzeigen; und
- bei dem Bestimmen ein Gewichten von jedem von dem Alterswert, dem Probenspiegel von Albumin und dem Probenspiegel von Kreatinin höher als den Probenspiegel einer glomerulären Filtrationsrate.

12. Verfahren nach mindestens einem der Ansprüche 1 bis 10, wobei der Risikofaktor nach der Gleichung

$$P_{CKD} = \frac{e^{P_{CKD\_Pred}}}{1+e^{P_{CKD\_Pred}}+e^{P_{Death\_Pred}}}$$

bestimmt wird und wobei

- $P_{CKD}$ der Risikofaktor ist;
- $P_{CKD\_Pred}$ = $C_{CKD1}$ · Alter + $C_{CKD2}$ · Kreatinin + $C_{CDK3}$ · Albumin + $C_{CKD4}$;
- $P_{Death\_Pred}$ = $C_{Death1}$ · Alter + $C_{Death2}$ · Kreatinin + $C_{Death3}$ · Albumin + $C_{Death4}$;
- Alter das Alter des Subjektes ist;
- Kreatinin ein Probenspiegel von Kreatinin für das Subjekt ist;
- Albumin ein Probenspiegel von Albumin für das Subjekt ist; und
- $C_{CKD1}$, $C_{CKD2}$, $C_{CKD3}$, $C_{CKD4}$, $C_{Death1}$, $C_{Death2}$, $C_{Death3}$ und $C_{Death4}$ Konstanten sind.

13. Verfahren nach mindestens einem der Ansprüche 1 bis 11, wobei der Risikofaktor nach der Gleichung

$$P'_{CKD} = \frac{e^{P'_{CKD\_Pred}}}{1+e^{P'_{CKD\_Pred}}+e^{P'_{Death\_Pred}}}$$

bestimmt wird und wobei

- $P'_{CKD}$ der Risikofaktor ist;
-

$P'_{CKD\_Pred}$ = $C'_{CKD1}$ · Alter + $C'_{CKD2}$ · Kreatinin + $C'_{CDK3}$ · Albumin + $C'_{CKD4}$ + $C'_{CKD5}$ · eGFR;

-

$P'_{Death\_Pred}$ = $C'_{Death1}$ · Alter + $C'_{Death2}$ · Kreatinin + $C'_{Death3}$ · Albumin + $C'_{Death4}$ + $C'_{Death5}$ · eGFR;

- Alter das Alter des Subjektes ist;
- Kreatinin ein Probenspiegel von Kreatinin für das Subjekt ist;
- Albumin ein Probenspiegel von Albumin für das Subjekt ist;
- eGFR ein Probenspiegel der geschätzten glomerulären Filtrationsrate für das Subjekt ist; und
- $C'_{CKD1}$, $C'_{CKD2}$, $C'_{CKD3}$, $C'_{CKD4}$, $C'_{CKD5}$, $C'_{Death1}$, $C'_{Death2}$, $C'_{Death3}$, $C'_{Death4}$ und $C'_{Death5}$ Konstanten sind.

**14.** Computer-implementiertes Verfahren zum Screening eines Subjektes auf das Risiko einer chronischen Nierenerkrankung (CKD) in einem Datenverarbeitungssystem, das einen Prozessor und einen nicht-transitorischen Speicher aufweist, der ein Programm speichert, das den Prozessor veranlasst, Folgendes auszuführen:

- Empfangen von Markerdaten, die für eine Mehrzahl von Markerparametern für ein Subjekt kennzeichnend sind, wobei die derartige Mehrzahl von Markerparametern für das Subjekt für einen Messzeitraum einen Alterswert, einen Probenspiegel von Albumin und einen Probenspiegel von Kreatinin anzeigen; und
- Bestimmen eines Risikofaktors, der auf das Risiko, an CKD zu erkranken, für das Subjekt aus der Mehrzahl von Markerparametern hinweist, wobei das Bestimmen Folgendes umfasst

- Gewichten des Alterswertes höher als den Probenspiegel von Albumin, und
- Gewichten des Probenspiegels von Kreatinin höher als den Probenspiegel von Albumin.

**15.** Verfahren zum Screening eines Subjektes auf das Risiko einer chronischen Nierenerkrankung (CKD), umfassend

- Empfangen von Markerdaten, die für eine Mehrzahl von Markerparametern kennzeichnend sind, wobei die derartige Mehrzahl von Markerparametern Folgendes anzeigt

- einen Alterswert für das Subjekt,
- einen Probenspiegel von Kreatinin für einen Messzeitraum, und
- einen Probenspiegel von Albumin für einen Messzeitraum; und

- Bestimmen eines Risikofaktors, der auf das Risiko, an CKD zu erkranken, für das Subjekt aus der Mehrzahl von Markerparametern hinweist, wobei das Bestimmen Folgendes umfasst

- Gewichten des Alterswertes höher als den Probenspiegel von Albumin, und
- Gewichten des Probenspiegels von Kreatinin höher als den Probenspiegel von Albumin,

wobei

- mindestens einer von dem Probenspiegel von Kreatinin und dem Probenspiegel von Albumin einen verallgemeinerten Wert von Probenspiegeln für eine Referenzgruppe von Subjekten, welche das Subjekt nicht umfasst, für einen entsprechenden Messzeitraum von jedem Subjekt der Referenzgruppe von Subjekten anzeigt.

## Revendications

**1.** Procédé de dépistage du risque de néphropathie chronique (CKD) chez un sujet, comprenant

- la réception de données de marqueurs indiquant une pluralité de paramètres de marqueurs pour un sujet, cette pluralité de paramètres de marqueurs indiquant, pour le sujet pour une période de mesure, une valeur d'âge, un niveau de créatinine d'échantillon, et un niveau d'albumine d'échantillon ; et
- la détermination d'un facteur de risque indiquant le risque que le sujet souffre de CKD à partir de la pluralité de paramètres de marqueurs, dans lequel la détermination comprend

- l'application à la valeur d'âge d'une pondération supérieure à celle appliquée au niveau d'albumine d'échantillon, et
- l'application au niveau de créatinine d'échantillon d'une pondération supérieure à celle appliquée au niveau d'albumine d'échantillon.

**2.** Procédé selon la revendication 1, comprenant en outre la pluralité de paramètres de marqueurs indiquant, pour le sujet, un niveau de créatinine d'échantillon sanguin.

**3.** Procédé selon la revendication 1 ou 2, comprenant en outre la pluralité de paramètres de marqueurs indiquant, pour le sujet, un niveau d'albumine d'échantillon sanguin.

**4.** Procédé selon au moins l'une quelconque des revendications précédentes, dans lequel le sujet est un patient souffrant de diabète.

**5.** Procédé selon au moins l'une quelconque des revendications précédentes, dans lequel la période de mesure est limitée à deux ans.

**6.** Procédé selon au moins l'une des revendications précédentes, dans lequel le sujet n'a pas été diagnostiqué comme souffrant de diabète à la fin de la période de mesure.

**7.** Procédé selon la revendication 4 ou 5, dans lequel la période de mesure s'étend après un diagnostic de diabète chez le sujet, au moins en partie.

**8.** Procédé selon au moins l'une des revendications précédentes, dans lequel le facteur de risque indique le risque que le sujet souffre de CKD au cours d'une période de prédiction de trois ans à partir de la fin de la période de mesure.

**9.** Procédé selon au moins l'une des revendications précédentes, dans lequel la détermination comprend en outre l'application à l'âge d'une pondération supérieure à celle appliquée au niveau de créatinine d'échantillon.

**10.** Procédé selon au moins l'une des revendications précédentes, dans lequel la réception comprend la réception de données de marqueurs indiquant une pluralité de paramètres de marqueurs pour un sujet ayant un niveau d'HbA1c d'échantillon inférieur à 6,5 %.

**11.** Procédé selon au moins l'une des revendications précédentes, comprenant en outre

  - la pluralité de paramètres de marqueurs indiquant, pour le sujet, un niveau de débit de filtration glomérulaire d'échantillon ; et
  - durant la détermination, l'application à chacun de la valeur d'âge, du niveau d'albumine d'échantillon, et du niveau de créatinine d'échantillon d'une pondération supérieure à celle du niveau de débit de filtration glomérulaire d'échantillon.

**12.** Procédé selon au moins l'une des revendications 1 à 10, dans lequel le facteur de risque est déterminé conformément à l'équation

$$P_{CKD} = \frac{e^{P_{CKD\_Pred}}}{1 + e^{P_{CKD\_Pred}} + e^{P_{Death\_Pred}}},$$

et dans lequel

  - $P_{CKD}$ est le facteur de risque ;
  - $P_{CKD\_Pred} = cC_{KD1} \cdot$ âge $+ c_{CKD2} \cdot$ créatinine $+ c_{CKD3} \cdot$ albumine $+ c_{CKD4}$ ;
  - $P_{Death\_Pred} = c_{Death1} \cdot$ âge $+ c_{Death2} \cdot$ créatinine $+ c_{Death3} \cdot$ albumine $+ c_{Death4}$ ;
  - âge est l'âge du sujet ;
  - créatinine est un niveau de créatinine d'échantillon pour le sujet ;
  - albumine est un niveau d'albumine d'échantillon pour le sujet ; et
  - $c_{CKD1}$, $c_{CKD2}$, $c_{CKD3}$, $c_{CKD4}$, $c_{Death1}$, $c_{Death2}$, $c_{Death3}$, et $c_{Death4}$ sont des constantes.

**13.** Procédé selon au moins l'une des revendications 1 à 11, dans lequel le facteur de risque est déterminé selon l'équation

$$P'_{CKD} = \frac{e^{P'_{CKD\_Pred}}}{1 + e^{P'_{CKD\_Pred}} + e^{P'_{Death\_Pred}}},$$

et dans lequel

  - $P'_{CKD}$ est le facteur de risque ;

$$P'_{CKD\_Pred} = c'_{CKD1} \cdot \text{âge} + c'_{CKD2} \cdot \text{créatinine} + c'_{CKD3} \cdot \text{albumine} + c'_{CKD4} + c'_{CKD5} \cdot \text{eGFR} ;$$

$$P'_{Death\_Pred} = c'_{Death1} \cdot \text{âge} + c'_{Death2} \cdot \text{créatinine} + c'_{Death3} \cdot \text{albumine} + c'_{Death4} + c'_{Death5} \cdot \text{eGFR} ;$$

- âge est l'âge du sujet ;
- créatinine est un niveau de créatinine d'échantillon pour le sujet ;
- albumine est un niveau d'albumine d'échantillon pour le sujet ;
- eGFR est un niveau de débit de filtration glomérulaire estimé d'échantillon pour le sujet ; et
- $c'_{CKD1}$, $c'_{CKD2}$, $c'_{CKD3}$, $c'_{CKD4}$, $c'_{CKD5}$, $c'_{Death1}$, $c'_{Death2}$, $c'_{Death3}$, $c'_{Death4}$ et $c'_{Death5}$ sont des constantes.

14. Procédé mis en oeuvre par ordinateur de dépistage chez un sujet du risque de néphropathie chronique (CKD) dans un système de traitement de données ayant un processeur et une mémoire non temporaire stockant un programme amenant le processeur à exécuter :

- la réception de données de marqueurs indiquant une pluralité de paramètres de marqueurs pour un sujet, cette pluralité de paramètres de marqueurs indiquant, pour le sujet pour une période de mesure, une valeur d'âge, un niveau d'albumine d'échantillon, et un niveau de créatinine d'échantillon ; et
- la détermination d'un facteur de risque indiquant le risque que le sujet souffre de CKD à partir de la pluralité de paramètres de marqueurs, dans lequel la détermination comprend

- l'application à la valeur d'âge d'une pondération supérieure à celle appliquée au niveau d'albumine d'échantillon, et
- l'application au niveau de créatinine d'échantillon d'une pondération supérieure à celle appliquée au niveau d'albumine d'échantillon.

15. Procédé de dépistage chez un sujet du risque de néphropathie chronique (CKD), comprenant

- la réception de données de marqueurs indiquant une pluralité de paramètres de marqueurs, cette pluralité de paramètres de marqueurs indiquant

- une valeur d'âge pour le sujet,
- un niveau de créatinine d'échantillon pour une période de mesure, et
- un niveau d'albumine d'échantillon pour une période de mesure ; et

- la détermination d'un facteur de risque indiquant le risque que le sujet souffre de CKD à partir de la pluralité de paramètres de marqueurs, dans lequel la détermination comprend

- l'application à la valeur d'âge d'une pondération supérieure à celle appliquée au niveau d'albumine d'échantillon, et
- l'application au niveau de créatinine d'échantillon d'une pondération supérieure à celle appliquée au niveau d'albumine d'échantillon,

dans lequel

- au moins l'un du niveau de créatinine d'échantillon et du niveau d'albumine d'échantillon indique une valeur généralisée des niveaux d'échantillon pour un groupe de sujets de référence ne comprenant pas le sujet, pour une période de mesure respective de chaque sujet du groupe de sujets de référence.

Fig. 1

Fig. 2

Fig. 3

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **UNGER, J. ; SCHWARTZ, Z.** Diabetes Management in Primary Care. Lippincott Williams & Wilkens, 2013
- **GLASSOCK, R.J. et al.** The global burden of chronic kidney disease: estimates, variability and pitfalls. *Nat Rev Nephrol,* 2017, vol. 13, 104-114
- **PLATINGA, L.C. et al.** Awareness of chronic kidney disease among patients and providers. *Adv Chronic Kidney Dis,* 2010, vol. 17, 225-236
- **FIORETTO, P. et al.** Residual microvascular risk in diabetes: unmet needs and future directions. *Nat Rev Endocrinol,* 2010, vol. 6, 19-25
- **DUNKLER, D. et al.** Risk Prediction for Early CKD in Type 2 Diabetes. *Clin J Am Soc Nephrol,* 2015, vol. 10, 1371-1379
- **VERGOUWE, Y. et al.** Progression to microalbuminuria in type 1 diabetes: development and validation of a prediction rule. *Diabetologia,* 2010, vol. 53, 254-262
- **KEANE, W.F. et al.** Risk Scores for Predicting Outcomes in Patients with Type 2 Diabetes and Nephropathy: The RENAAL Study. *Clin J Am Soc Nephrol,* 2006, vol. 1, 761-767
- **JARDINE, M.J. et al.** Prediction of Kidney-Related Outcomes in Patients With Type 2 Diabetes. *Am J Kidney Dis.,* 2012, vol. 60, 770-778
- **ADLER PEROTTE et al.** Risk prediction for chronic kidney disease progression using heterogeneous electronic health record data and time series analysis. *Journal of the American Medical Informatics Association,* 20 April 2015, vol. 22 (4), 872-880
- **PAOLO FRACCARO et al.** An external validation of models to predict the onset of chronic kidney disease using population-based electronic health records from Sal-ford, UK. *BMC Medicine,* 12 July 2016, vol. 14 (1

- **JUSTIN B. ECHOUFFO-TCHEUGUI et al.** Risk Models to Predict Chronic Kidney Disease and Its Progression: A Systematic Review. *Plos Medicine,* 20 November 2012, vol. 9 (11), e1001344
- **KAELBER, D.C. et al.** Patient characteristics associated with venous thromboembolic events: a cohort study using pooled electronic health record data. *J Am Med Inform Assoc,* 2012, vol. 19, 965-972
- **MCDONALD, C. J. et al.** The Indiana Network for Patient Care: a working local health information infrastructure. *Health Affairs,* 2005, vol. 24, 1214-1220
- **BURSAC, Z. et al.** Purposeful selection of variables in logistic regression. *Source code for biology and medicine,* 2008, vol. 3, 17
- **HOSMER JR., D.W. et al.** Applied logistic regression. John Wiley & Sons, 2013, vol. 398
- **SWETS, J.A.** Measuring the accuracy of diagnostic systems. *Science,* 1988, vol. 240, 1285-1293
- **BRADLEY, A.P.** The use of the area under the ROC curve in the evaluation of machine learning algorithms. *Pattern Recognition,* 1997, vol. 30, 1145-1159
- The Diabetes Control and Complications Trial Research Group. The effect of intensive treatment of diabetes on the development and progression of long-term complications in insulin-dependent diabetes mellitus. *N Engl J Med,* 1993, vol. 329, 977-986
- **VAN RIJSBERGEN, C.J.** Information Retrieval. Butterworth-Heinemann, 1979